# FASCICULE DE BREVET EUROPEEN

(11) **EP 2 252 263 B1**
(45) Date de publication et mention de la délivrance du brevet: **13.07.2011**
(21) Numéro de dépôt: 09706112.1
(22) Date de dépôt: 29.01.2009
(51) Int. Cl.: A61K 9/08, A61K 31/404, A61P 25/06

(54) **FORME GALENIQUE POUR L'ADMINISTRATION DE TRIPTANS PAR VOIE TRANS-MUQUEUSE BUCCALE**
GALENISCHE FORM ZUR ORALEN TRANSMUKOSALEN ZUFÜHRUNG VON TRIPTANEN
GALENIC FORM FOR THE ORAL TRANSMUCOSAL DELIVERY OF TRIPTANS

(30) Priorité: 30.01.2008 FR 0850571
(43) Date de publication de la demande: 24.11.2010
(73) Titulaire: Perovitch, Philippe, 33680 Le Temple (FR); Maury, Marc, 33160 Saint Medard en Jalles (FR)
(72) Inventeur: Perovitch, Philippe, 33680 Le Temple (FR); Maury, Marc, 33160 Saint Medard en Jalles (FR)
(74) Mandataire: CAPRI
(86) Numéro de dépôt international: PCT/FR2009/050134
(87) Numéro de publication internationale: WO 2009/095621

(56) Documents cités:
- WO-A-2005/032518
- WO-A-2008/035020

## Description

La présente invention concerne une forme galénique pour l'administration systémique instantanée par voie trans-muqueuse buccale d'au moins un principe actif appartenant à la famille des Triptans, en particulier le Sumatriptan.

L'invention se rapporte également à un procédé d'obtention de cette forme galénique et à son utilisation.

Les Triptans sont des actifs pharmaceutiques utilisés pour le traitement des crises migraineuses, en particulier de l'Algie Vasculaire de la Face (AVF), forme aiguë de céphalée représentant une véritable urgence thérapeutique.

Le Sumatriptan est l'anti-migraineux majeur et de référence du marché, mais tous les Triptans possèdent la même nature physico-chimique et le même mode d'action vasculaire central. On connaît notamment le Zolmitriptan, l'Almotriptan, l'Elétriptan, le Naratriptan et le Frovatriptan.

Les Triptans ont la capacité de stimuler certains récepteurs de la sérotonine et plus précisément les récepteurs vasculaires à la 5-HydroxyTryptamine 1 (5-HT1). La stimulation de ces récepteurs entraîne une vasoconstriction centrale réduisant les phénomènes de vasodilatation et oedème à l'origine de la crise d'AVF.

On sait que les Triptans peuvent être administrés par voie intraveineuse en perfusion mais cette application est demeurée expérimentale du fait de la difficulté de son application thérapeutique courante, bien qu'une telle administration ait démontré un délai très court de réponse clinique efficace pour une très faible dose administrée.

En effet, ce mode d'administration, nécessitant du personnel dédié et l'utilisation de matériels spécifiques, est d'un coût élevé, est lourd pour le patient soumis à hospitalisation et n'est pas adapté au traitement des crises migraineuses. En conséquence, si les bénéfices de l'administration de Triptans par voie intraveineuse ont été cliniquement évalués et sont extrêmement satisfaisants, l'exploitation commerciale de ces formes galéniques spécifiques intraveineuses n'a jamais été entreprise.

Les crises étant imprévisibles, certains patients ont besoin d'un traitement journalier, si bien que seule une auto-administration est envisageable.

Il existe actuellement plusieurs modes d'auto-administration de Triptans.

Une première voie connue est l'administration sous forme de comprimés. Néanmoins les Triptans administrés par la voie entérale présentent un délai d'action d'environ deux heures, délai démesuré par rapport à l'attente de tout patient en souffrance.

Lorsqu'ils sont introduits dans le tube digestif et l'estomac, ces principes actifs subissent l'effet dit de « premier passage digestif », altérations et déperditions liées au milieu stomacal ou aux variations des physiologies intestinales, dont une stase stomacale, paralysie abondamment décrite comme constante chez les migraineux, laquelle réduit d'au moins 50% l'absorption de tout principe actif.

Ils sont ensuite soumis à un effet dit de « premier passage hépatique » qui provoque leur métabolisation et/ou leur dégradation plus ou moins intense, avec constitution de nombreux métabolites, pour la plupart inactifs ou toxiques provoquant des effets secondaires.

La dose de principe actif véritablement biodisponible est donc extrêmement faible : seule une part très résiduelle de la quantité administrée demeure valide pour produire l'effet pharmacologique attendu.

Ainsi, à titre d'exemple, administré par voie digestive, le Sumatriptan présente une biodisponibilité résiduelle réduite à moins de 14% de la dose administrée, avec simultanément un volume moyen de distribution théorique dans le corps humain de 200 litres et une demi-vie de 2 heures seulement quand la concentration plasmatique maximale n'est atteinte qu'après 1 à 1,5 heures.

On sait aussi que le début d'un effet clinique sur l'affection ne débute en moyenne qu'à partir de 30 minutes après la prise.

De fait, il apparaît deux problèmes majeurs.

Le premier problème est qu'il faut administrer une dose suffisante de Triptan au patient, tenant compte du statut pondéral du sujet, de l'absorption, de la métabolisation, de la dilution et de la dispersion de ce principe actif dans l'organisme, pour que la seule part résiduelle active atteigne les récepteurs vasculaires à la 5-HydroxyTryptamine 1 (5-HT1) et produise une efficacité pharmacologique.

Le second est le temps de latence incompressible lié à l'absorption, à la métabolisation et à la diffusion du principe actif dans l'organisme avant que la molécule agisse et que le patient en ressente les bienfaits.

L'administration de Triptans à l'aide de comprimés par voie digestive n'est donc pas appropriée ni au traitement rapidement efficace de la migraine, ni à l'urgence d'une céphalée cataclysmique comme l'Algie Vasculaire de la Face.

On connaît également d'autres voies possibles d'auto-administration des Triptans comme la voie per-muqueuse buccale, la voie per-muqueuse nasale et la voie injectable sous-cutanée.

La voie per-muqueuse buccale permet d'administrer des médicaments par franchissement passif des muqueuses sublinguales, jugales, gingivales, linguales, palatines ou pharyngées, puis passage dans les veines sub-linguales et distribution cardiaque et à la circulation générale, court-circuitant ainsi le passage digestif et le métabolisme hépatique.

Néanmoins, les formulations existantes, comme les comprimés orodispersibles de Zolmitriptan, ne sont pas satisfaisantes, notamment en raison du fait que les Triptans sont par nature insolubles dans les liquides biologiques comme la salive. Pour ces formes, si leur dispersibilité est orale, le principe actif insoluble est ensuite dégluti et subit le même sort métabolique digestif que celui des formes Triptans en comprimés, ce dont témoigne l'équivalence de biodisponibilité revendiquée par ces deux formes - orodispersible et comprimé - dans leurs mentions légales.

C'est le cas également des sprays et capsules de gélatines à mordre destinés à une administration par voie transmuqueuse, décrits dans la demande WO-2005/032518. Ces formulations présentent des caractéristiques qui ne sont pas satisfaisantes en termes de précision d'administration, de rendements d'absorption et de biodisponibilité assurée des dosages administrés. Il s'agit de formulations liquides complexes qui comportent une association d'ingrédients multiples destinés à solubiliser et stabiliser des molécules de Sumatriptan, notamment Propylène Glycol, Polyéthylène Glycol, Acide benzoïque, etc. et à créer un état de viscosité très spécifique pour une distribution par spray. Lors de l'administration, la distribution dans la cavité buccale reste pourtant diffuse et aléatoire et dès réception des particules propulsées du spray, celles-ci sont instantanément mélangées à de la salive produite de manière réflexe et mécanique dans la cavité buccale. Ce mélange est généralement dégluti de manière automatique par le patient avant que le principe actif n'ait eu l'opportunité de franchir les muqueuses buccales pour passer dans le circuit vasculaire veineux. Seule une très faible fraction du principe actif formulé est donc directement disponible par passage per-muqueux, autour de 20 à 25% de la dose unitaire administrée et l'efficacité reste encore très éloignée de celle obtenue par voie intraveineuse ou sous-cutanée.

La voie nasale permet elle, une absorption per-muqueuse du principe actif par pulvérisation d'une forme liquidienne (spray). Dans le domaine des anti-migraineux, on connaît notamment des sprays nasaux constitués de Sumatriptan base en solution aqueuse à 4 excipients (Imigrane). Si cette voie nasale a montré un rendement dose/effet légèrement meilleur que la voie entérique, la concentration maximale de Sumatriptan est évaluée entre 13,1 et 14,14ng/mL de plasma, 30 minutes après l'administration d'une dose unique intra-nasale de 20mg. La biodisponibilité moyenne de la dose administrée n'est donc pas supérieure à 10%. Ce rendement insuffisant est en outre toujours aléatoire du fait du possible encombrement des voies nasales par des mucosités. Ainsi, ces formulations pour administration par voie nasale ne sont pas non plus satisfaisantes.

Il existe encore un autre mode d'administration, qui a été développé il y a une vingtaine d'années pour le Sumatriptan : la voie injectable sous-cutanée, à administration médicale ou infirmière ou en auto-injection. Des études ont montré que l'injection sous-cutanée de Sumatriptan à un dosage unique de 6mg procure 75% de rémission de la crise d'Algie Vasculaire de la Face dans un délai moyen de 15 minutes.

Toutefois ce mode d'administration, qui représente un coût unitaire élevé, nécessite l'utilisation d'un matériel spécifique, un auto-injecteur, si bien qu'il se révèle pour un patient en crise avérée, peu ergonomique, douloureux et beaucoup plus invasif que la simple prise de comprimés ou l'utilisation d'un spray.

En outre, bien qu'il s'agisse de la forme exclusive d'auto-administration la plus efficace existant actuellement en traitement de la crise, elle reste encore très éloignée de l'efficacité obtenue par voie intraveineuse qui produit 90% de rémissions après 3 à 4 minutes.

Il subsiste donc un besoin pour une formulation galénique simple d'utilisation, moins onéreuse, facilement disponible et peu invasive, permettant d'administrer une quantité immédiatement biodisponible de Triptan, de façon à pouvoir traiter très rapidement et efficacement des symptômes douloureux ou des troubles invalidants des crises migraineuses.

C'est ce à quoi répond la présente invention en proposant une forme galénique très spécifique permettant de garantir l'administration instantanée par voie trans-muqueuse buccale d'au moins un principe actif de la famille des Triptans, comprenant au moins :
- ledit principe actif sous forme base et sous forme de sel, et
- une solution hydroalcoolique titrant au moins 15 degrés d'alcool,
ledit principe actif étant présent en état de dissolution stable et complète dans la solution hydroalcoolique.

L'invention propose également un procédé de préparation ainsi que l'utilisation de cette formulation pour le traitement ou la prévention des symptômes douloureux ou des troubles invalidants des crises migraineuses.

Avantageusement la formulation selon l'invention permet le passage per-muqueux instantané et complet d'une préparation thérapeutique à base de Triptans, en limitant toute dilution salivaire et déglutition des molécules de Triptans qui sont délivrées quasi-instantanément au système vasculaire pour une distribution de la totalité du dosage aux récepteurs cérébraux 5-HT1 de la sérotonine.

D'autres caractéristiques et avantages ressortiront de la description qui va suivre de l'invention.

Selon un premier aspect, l'invention a donc pour objet une formulation pour l'administration par voie trans-muqueuse buccale d'au moins un principe actif de la famille des Triptans, comprenant au moins :
- ledit principe actif sous forme base et sous forme de sel, et
- une solution hydroalcoolique titrant au moins 15 degrés d'alcool,
ledit principe actif étant présent en état de dissolution stable et complète dans la solution hydroalcoolique, de façon à permettre une absorption rapide dudit principe actif à travers les muqueuses de la cavité buccale et/ou de l'oropharynx. Par « voie trans-muqueuse buccale », on entend tout franchissement passif d'une molécule lipophile ou amphiphile à travers les muqueuses linguales, sublinguales, gingivales, palatines, jugales, ou tout autres muqueuses constitutives de la cavité buccale et de l'oropharynx.

Par « état de dissolution stable et complète », on entend un état de dissolution restituant le principe actif à l'état moléculaire et faiblement ionisé dans son milieu de dissolution, état de dissolution prévenant toute éventualité d'une recristallisation inopportune.

Par « solution hydroalcoolique titrant X degrés d'alcool », on entend une solution présentant un degré d'alcool de X, correspondant au rapport entre le volume d'alcool pur (100°) contenu dans la solution hydroalcoolique et le volume total de cette solution. Le degré d'alcool de la solution hydroalcoolique varie en fonction du degré de l'alcool utilisé pour former la solution et du ratio eau/alcool de la solution. Par exemple pour un alcool initial à 100 degrés et un ratio eau/alcool 50/50, la solution hydroalcoolique titre 50 degrés d'alcool.

Le principe actif de la famille des Triptans est présent sous forme base et sous forme de sel, par exemple sous forme de Succinate, de Chlorhydrate ou de Sulfate. Préférentiellement le principe actif est présent entre 5 et 95 % sous forme base et entre 5 et 95% sous forme de sel. Encore plus préférentiellement, le principe actif est présent entre 5 et 40 % sous forme base et entre 60 et 95% sous forme de sel.

Selon un mode réalisation particulièrement adapté, le principe actif est le Sumatriptan.

De façon préférentielle, la forme galénique selon l'invention se présente sous forme d'une solution hydroalcoolique comprenant entre 15 et 85% d'alcool en masse et une teneur en eau comprise entre 15 et 85%. Encore plus préférentiellement, la formulation selon l'invention se présente sous forme d'une solution hydroalcoolique comprenant entre 30 et 85% d'alcool en masse et une teneur en eau comprise entre 15 et 70%.

La solution hydroalcoolique a un degré variable d'alcool d'au moins 15 degrés, préférentiellement compris entre 15 et 95 degrés, encore plus préférentiellement entre 20 et 70 degrés, et idéalement autour de 45 degrés.

Avantageusement, la solution hydroalcoolique est le seul solvant utilisé dans la formulation selon l'invention.

En outre, l'alcool de la solution hydroalcoolique ne joue pas seulement le rôle de diluant, mais également celui de promoteur d'une absorption per-muqueuse accélérée, dont la vitesse croit en fonction de l'élévation du degré d'alcool utilisé.

Selon un mode de réalisation préféré de l'invention, la solution hydroalcoolique est réalisée à base d'eau et d'éthanol.

A titre illustratif, le coefficient de dissolution du sumatriptan dans l'éthanol permet d'obtenir une dissolution complète dudit principe actif à hauteur de 2mg, 4mg ou 6mg de Sumatriptan pour 0,75ml d'éthanol à 35%. Ce coefficient peut être modulé en fonction du degré d'alcool et du ratio eau/éthanol utilisé.

La forme galénique selon l'invention peut aussi comprendre un agent correcteur de pH.

Par agent correcteur de pH on entend tout agent acide ou tout agent basique n'altérant pas les caractères physico-chimiques du ou des principes actifs.

Préférentiellement, l'agent correcteur de pH est choisi parmi les carbonates et bicarbonates de sodium, les phosphates monosodique ou disodique, la triéthanolamine, la soude (NaOH) et la potasse (KOH) mais aussi des agents acides Chlorhydrique, Sulfurique, Phosphorique, Citrique, Malique, Lactique, Succinique et/ou Butyrique.

Préférentiellement le pH de la formulation selon l'invention est compris entre 4,0 et 9,0, encore plus préférentiellement entre 4,0 et 8,0.

La forme galénique selon l'invention permet au principe actif de franchir passivement les muqueuses buccales dans un délai inférieur à 30 secondes après administration. Ce délai d'absorption très rapide permet de prévenir toute stagnation de la solution et du principe actif dans l'atmosphère buccale ainsi que leur mélange inopportun avec de la salive susceptible de les altérer, ce qui introduirait une rupture dans la continuité et la stabilité de la dissolution du ou des principes actifs. Ce court délai permet également de prévenir toute déglutition réflexe de la solution et du principe actif qu'elle contient.

Le passage trans-muqueux buccal du principe actif présenté en état de dissolution selon l'invention du côté de la membrane épithéliale externe, constituée de structures phospho-lipidiques qui absorbent passivement les molécules lipophiles par affinité élective, est basé sur un appel osmotique vers l'autre côté de ladite membrane, auquel participent ensemble la concentration en principe actif dissous et celle de la solution alcoolique considérée. L'appel osmotique est d'autant plus vivace et puissant que le degré d'alcool qui sert de promoteur d'absorption est élevé. Dans le cas particulier du Sumatriptan, selon l'invention, un degré d'alcool adapté est compris entre 15° et 95°, préférentiellement entre 20° et 70°. Ceci permet d'assurer simultanément l'obtention et le réglage du meilleur coefficient de dissolution et de stabilisation du Sumatriptan ainsi que la promotion de son passage per-muqueux dans un délai d'une dizaine de secondes. Un mode de réalisation particulièrement adapté correspond à 0,75ml de solution hydro alcoolique à un degré d'alcool de 45° pour 2mg, 4mg ou 6mg de Sumatriptan.

Les muqueuses de la bouche possèdent un réseau de micro-vaisseaux très dense, quasi-spongieux, si bien que les molécules, tant de solvant alcoolique que de principe actif dissous, qui franchissent les pores lipophiles de la membrane épithéliale, sont instantanément capturées par la micro-circulation sanguine et collectées vers les veines sublinguales et du plancher de la bouche. Ce phénomène est accentué par la présence de l'alcool qui provoque une vasodilatation et un accroissement du débit micro-vasculaire local des muqueuses.

Du fait de ce débit circulatoire élevé, localement accru par l'alcool, il n'y a donc jamais d'équilibre de part et d'autre de la membrane : la concentration dans la bouche reste toujours plus importante, jusqu'à épuisement du mécanisme par défaut de molécules à absorber.

Ainsi, à la différence notoire de toutes les autres formes dites « sub-linguales », la totalité de l'alcool et du principe actif qui s'y trouve dissous selon l'invention, passe à travers la muqueuse buccale en quelques secondes.

L'utilisation de la forme galénique selon l'invention permet d'administrer passivement une dose de Triptan immédiatement absorbée dès que déposée au contact de la muqueuse, pour être distribuée dans l'instant par voie vasculaire, sans aucun délai pour son action pharmacologique et sans subir les effets préalables majeurs de passage digestif et hépatique. La forme galénique selon l'invention permet donc une immédiateté d'absorption tissulaire complète des molécules de Triptan, puis leur distribution dans la circulation centrale de l'organisme, générant une réponse pharmacologique rapide de type « flash ».

Par exemple, avec une forme galénique selon l'invention réalisée à partir de 2mg de Sumatriptan solubilisé dans 0,75ml d'alcool éthylique à 45°, on peut administrer quasi-instantanément et passivement une dose très significative de Sumatriptan, 2mg, qui correspond à la dose normalement administrée par voie intra-veineuse, soit moins d'un vingt-cinquième de la dose usuellement administrée par voie orale (50mg).

La solution hydroalcoolique titrant au moins 15° d'alcool selon l'invention, présente également l'avantage de solubiliser les molécules de Triptans bien qu'elles soient lipophiles, ce qui autorise leur absorption per-muqueuse spontanée et de protéger la formulation pharmaceutique vis-à-vis d'une contamination microbiologique sans devoir introduire d'agent(s) de conservation anti-microbien(s).

Ainsi, la solution hydroalcoolique selon l'invention possède une quadruple compétence :
- elle joue le rôle de solvant du principe actif de la famille des Triptans, molécules lipophiles et de faible poids moléculaire,
- elle active le passage per-muqueux de ce principe actif dissous ainsi présenté à l'état moléculaire au niveau de la membrane lipophile,
- le degré d'alcool accroît doublement la vitesse d'absorption muqueuse, par effet osmotique et en suscitant une vasodilatation micro-vasculaire réflexe, qui accélère le débit micro-circulatoire local, et
- elle est son propre agent de stabilité ce qui évite l'utilisation d'additifs conventionnels.

Avantageusement, la présente invention offre une grande simplicité de réalisation et une très bonne stabilité galénique : la solution eau/alcool garantit la solubilisation du principe actif tout en supprimant la plupart des excipients utilisés dans les formes pharmaceutiques usuelles. Elle permet donc à la fois de réduire les coûts de fabrication et de diminuer les risques d'intolérance et les possibles interactions entre principe actif et excipients.

De façon singulière, les délais d'action de la forme galénique selon l'invention sont très courts, comparés aux lenteurs d'absorptions des médicaments à base de Triptans existants destinés à une auto-administration.

La délivrance pharmacologique quasi-instantanée permet à un patient de s'administrer lui-même un produit pour un effet équivalent à l'efficacité d'une injection intraveineuse de Triptan en flash dans la circulation.

Il s'agit d'une voie d'administration bien meilleure en termes de simplicité et de disponibilité d'administration non traumatique mais aussi de coût unitaire et thérapeutique comparée à tous les modes d'administration de Triptan existants. Le gain en termes de rapport dose/effet est considérable puisque l'on n'utilise plus que 4 à 8% de la dose pondérale de référence par voie orale, soit entre 92 et 96% de dose en moins pour un effet thérapeutique obtenu dans ce cas sans délai.

En outre, les molécules de Triptan ne rencontrant pas d'obstacle significatif pour leur distribution instantanée dans la circulation artérielle cérébrale qu'elles gagnent en quelques secondes, la dose de base administrée est réduite, proche de la dose utile pour exercer l'activité pharmacologique requise. Cette dose est bien entendu dépendante de l'effet recherché. Elle est préférentiellement comprise entre 0,5mg et 6mg pour le Sumatriptan, pour des volumes d'administration c'est-à-dire des volumes de solution hydroalcoolique, inférieurs à 5ml, préférentiellement variant de 0,5ml à 1ml.

Par ailleurs, la muqueuse buccale disposant d'une surface totale d'absorption extrêmement large, démultipliée par son caractère de tissu villeux plissé, l'administration de la forme galénique selon l'invention est dépourvue d'un quelconque risque de déglutition intempestive ou de fausse route. En effet, elle permet un passage per-muqueux extrêmement rapide qui prévient toute dissolution salivaire ou déglutition du principe actif administré, avec l'avantage de ne pas déstabiliser les muqueuses, par exemple avec des dérivés tensioactifs, comme c'est le cas de certaines formulations existantes à vocation « sublinguale».

De même, les effets de l'alcool sont insignifiants. A titre d'exemple, 0,75 ml d'Ethanol à 35° ne sauraient produire qu'une alcoolémie circulante inférieure à 0,004g par litre de sang, soit 1/125ième des tolérances légales en France de 0,5g par litre.

Un procédé de fabrication de la forme galénique selon l'invention, particulièrement adapté, comprend les étapes suivantes :
- introduire dans de l'alcool au moins un principe actif de la famille des Triptans sous forme base,
- agiter la préparation jusqu'à dissolution complète dudit principe actif,
- ajouter de l'eau purifiée à la préparation, puis ledit principe actif sous forme de sel,
- agiter la préparation jusqu'à dissolution complète, et
- ajouter de l'eau si nécessaire pour compléter au volume désiré.

Selon un mode de réalisation préféré le procédé comprend les étapes suivantes :
- introduire dans de l'alcool sous agitation au moins un principe actif de la famille des Triptans sous forme base, préférentiellement du Sumatriptan base,
- agiter la préparation, préférentiellement durant 10 à 60 minutes, jusqu'à dissolution complète dudit principe actif,
- ajouter de l'eau purifiée à la préparation, puis ledit principe actif sous forme de sel, préférentiellement du Succinate de Sumatriptan,
- agiter la préparation, préférentiellement durant 10 à 60 minutes, jusqu'à dissolution complète,
- ajouter de l'eau si nécessaire pour compléter au volume désiré,
- ajouter des agents correcteurs de pH pour obtenir un pH physiologiquement compatible compris entre 4,0 et 9,0, préférentiellement entre 4,0 et 8,0,
- filtrer la préparation, et
- répartir le soluté obtenu dans des contenants adaptés de moins de 5ml de volume, préférentiellement des flacons en verre brun ou polymère opaque de 2ml, garantissant la stabilité du produit.

La présente invention peut être utilisée pour l'administration systémique instantanée à doses réduites et utiles de Triptan, notamment de Sumatriptan.

En particulier la forme galénique selon la présente invention peut être utilisée pour la réalisation d'un médicament pour le traitement de la migraine en général, et des crises d'AVF en particulier. Un tel médicament présente une activité thérapeutique anti-migraineuse dans un délai très bref et à des doses très réduites par rapport aux doses traditionnelles.

La formulation selon l'invention, correspondant à un très faible volume liquidien, est très facile à administrer. Un patient peut aisément la déposer dans sa bouche au contact direct d'une zone muqueuse précise, de surface réduite. De préférence le patient doit déposer la formulation selon l'invention au niveau d'un territoire muqueux à l'abri des sécrétions salivaires, par exemple la gouttière jugale, délimitée d'une part par la couronne gingivale inférieure et externe et d'autre part par la paroi muqueuse des faces inférieures et internes des joues et lèvre inférieure. Ce canal représente en moyenne un réservoir clos d'environ 18 cm de long et de 1 à 1,5cm de profondeur, soit une surface d'absorption muqueuse de 35 à 55 cm².

Selon un dernier aspect, la formulation selon l'invention nécessite un conditionnement industriel spécifique, afin de permettre son utilisation sécurisée, simple et ergonomique et de prévenir la dégradation du principe actif au contact de l'air.

Un mode de réalisation particulier consiste à utiliser un conditionnement, préférentiellement de petite taille, plastique ou métalloplastique souple ou en verre, opaque rempli sous atmosphère inerte tel que de l'azote, pour la protection de la stabilité de la composition et l'imperméabilité à l'oxygène et aux rayonnements. Ces conditionnements garantissent la dissolution et la stabilité dans le temps des principes actifs dissous en solution hydroalcoolique selon l'invention.

Préférentiellement ces conditionnements comportent une canule permettant le dépôt précis de la solution selon l'invention au contact d'une zone muqueuse adéquate.

Pour le confort d'utilisation par le patient, pour un transport aisé, on peut préférentiellement recourir à des emballages sous forme d'étuis étanches spécifiques. Encore plus préférentiellement, la forme galénique selon l'invention est conditionnée dans des emballages unidoses de 0,5 à 5 ml, susceptibles de fournir une dose adéquate de principe actif.

De façon avantageuse, ce conditionnement est facile à transporter et permet une utilisation aisée de la forme galénique à tout moment de la journée.

On peut citer plusieurs exemples de formulation du Sumatriptan selon l'invention, d'un volume de 0,75ml, particulièrement adaptée pour produire une efficacité au niveau cérébral dans un délai de quelques minutes seulement :

### Formulation 1:

| | | | |
|---|---|---|---|
| - Sumatriptan : | | | 6,0mg |
| introduit sous forme de : | | | |
| | Sumatriptan base | 1,20mg | |
| | Sumatriptan Succinate | 6,72mg | |
| - alcool éthylique 95% V :V : | | | 0,3ml |
| - Eau purifiée : | | | qsp 0,75ml |

Il est à noter que la quantité de sumatriptan dans 6,72mg de sumatriptan succinate est de 4,8mg.

Cet exemple de formulation peut être obtenu par la mise en oeuvre du procédé décrit en suivant pour un lot de 1000 doses, soit 0,75 L.

Dans une cuve en acier inoxydable introduire 0,3 L d'éthanol 95% V/V et 1,20g de sumatriptan base.

A l'aide d'un agitateur à hélice agiter la préparation jusqu'à dissolution complète du Sumatriptan base.

Ajouter 0,450 L d'eau purifiée au mélange, puis 6,72g de Sumatriptan Succinate. A l'aide d'un agitateur à hélice, agiter la préparation jusqu'à obtention d'une suspension homogène et dissolution complète.

Filtrer la préparation sur un filtre en polypropylène ou équivalent de 5µm de porosité et répartir la préparation dans des flacons monodoses de 0,75 mL.

### Formulation 2 :

| | | | |
|---|---|---|---|
| - Sumatriptan : | | | 4,0mg |
| introduit sous forme de : | | | |
| | Sumatriptan base | 0,8mg | |
| | Sumatriptan Succinate | 4,48mg | |
| - alcool éthylique 95% V :V : | | | 0,3ml |
| - Eau purifiée : | | | qsp 0,75ml |

Il est à noter que la quantité de sumatriptan dans 4,48mg de sumatriptan succinate est de 3,2mg.

Cet exemple de formulation peut être obtenu par la mise en oeuvre du procédé décrit en suivant pour un lot de 1000 doses, soit 0,75 L.

Dans une cuve en acier inoxydable introduire 0,3 L d'éthanol 95% V/V et 0,8g de Sumatriptan base.

A l'aide d'un agitateur à hélice agiter la préparation jusqu'à dissolution complète du Sumatriptan base.

Ajouter 0,450 L d'eau purifiée au mélange, puis 4,48g de Sumatriptan Succinate. A l'aide d'un agitateur à hélice, agiter la préparation jusqu'à obtention d'une suspension homogène et dissolution complète.

Filtrer la préparation sur un filtre en polypropylène ou équivalent de 5µm de porosité et répartir la préparation dans des flacons monodoses de 0,75 mL.

### Formulation 3 :

| | | | |
|---|---|---|---|
| - Sumatriptan : | | | 2,0mg |
| introduit sous forme de : | | | |
| | Sumatriptan base | 0,4mg | |
| | Sumatriptan Succinate | 2,24mg | |
| - alcool éthylique 95% V :V : | | | 0,3ml |
| - Eau purifiée: | | | qsp 0,75ml |

Il est à noter que la quantité de sumatriptan dans 2,24mg de sumatriptan succinate est de 1,6mg.

Cet exemple de formulation peut être obtenu par la mise en oeuvre du procédé décrit en suivant pour un lot de 1000 doses, soit 0,75 L.

Dans une cuve en acier inoxydable introduire 0,3 L d'éthanol 95% V/V et 0,40g de Sumatriptan base.

A l'aide d'un agitateur à hélice agiter la préparation jusqu'à dissolution complète du Sumatriptan base.

Ajouter 0,450 L d'eau purifiée au mélange, puis 2,24g de Sumatriptan Succinate. A l'aide d'un agitateur à hélice, agiter la préparation jusqu'à obtention d'une suspension homogène et dissolution complète.

Filtrer la préparation sur un filtre en polypropylène ou équivalent de 5µm de porosité et répartir la préparation dans des flacons monodoses de 0,75 mL.

### Formulation 4 :

| | | | |
|---|---|---|---|
| - Sumatriptan : | | | 1,0mg |
| introduit sous forme de : | | | |
| | Sumatriptan base | 0,20mg | |
| | Sumatriptan Succinate | 1,12mg | |
| - alcool éthylique 95% V :V : | | | 0,3ml |
| - Eau purifiée: | | | qsp 0,75ml |

Il est à noter que la quantité de sumatriptan dans 1,12mg de sumatriptan succinate est de 0,8mg.

Cet exemple de formulation peut être obtenu par la mise en oeuvre du procédé décrit en suivant pour un lot de 1000 doses, soit 0,75 L.

Dans une cuve en acier inoxydable introduire 0,3 L d'éthanol 95% V/V et 0,20g de Sumatriptan base.

A l'aide d'un agitateur à hélice agiter la préparation jusqu'à dissolution complète du Sumatriptan base.

Ajouter 0,450 L d'eau purifiée au mélange, puis 1,12g de Sumatriptan succinate. A l'aide d'un agitateur à hélice, agiter la préparation jusqu'à obtention d'une suspension homogène et dissolution complète.

Filtrer la préparation sur un filtre en polypropylène ou équivalent de 5µm de porosité et répartir la préparation dans des flacons monodoses de 0,75 mL.

### Formulation 5 :

| | | | |
|---|---|---|---|
| - Sumatriptan : | | | 1,0mg |
| introduit sous forme de : | | | |
| | Sumatriptan base | 0,20mg | |
| | Sumatriptan Succinate | 1,12mg | |
| - alcool éthylique 95% V :V : | | | 0,3ml |
| - Eau purifiée : | | | qsp 0,75ml |
| - Hydroxyde de sodium : | | | qsp pH 7,5 |

Il est à noter que la quantité de sumatriptan dans 1,12mg de sumatriptan succinate est de 0,8mg.

Cet exemple de formulation peut être obtenu par la mise en oeuvre du procédé décrit en suivant pour un lot de 1000 doses, soit 0,75 L.

Dans une cuve en acier inoxydable introduire 0,3 L d'éthanol 95% V/V et 0,20g de Sumatriptan base.

A l'aide d'un agitateur à hélice agiter la préparation jusqu'à dissolution complète du Sumatriptan base.

Ajouter 0,450 L d'eau purifiée au mélange, puis 1,12g de Sumatriptan succinate. A l'aide d'un agitateur à hélice, agiter la préparation jusqu'à obtention d'une suspension homogène et dissolution complète.

Ajouter de l'hydroxyde de sodium pour ajuster le pH de la solution à 7,5.

Filtrer la préparation sur un filtre en polypropylène ou équivalent de 5µm de porosité et répartir la préparation dans des flacons monodoses de 0,75 mL.

### Formulation 6 :

| | | | |
|---|---|---|---|
| - Sumatriptan : | | | 0,75mg |
| introduit sous forme de : | | | |
| | Sumatriptan base | 0,15mg | |
| | Sumatriptan Succinate | 0,84mg | |
| - alcool éthylique 95% V :V : | | | 0,3ml |
| - Eau purifiée : | | | qsp 0,75ml |

Il est à noter que la quantité de sumatriptan dans 0,84mg de sumatriptan succinate est de 0,60mg.

Cet exemple de formulation peut être obtenu par la mise en oeuvre du procédé décrit en suivant pour un lot de 1000 doses, soit 0,75 L.

Dans une cuve en acier inoxydable introduire 0,3 L d'éthanol 95% V/V et 0,15g de Sumatriptan base.

A l'aide d'un agitateur à hélice agiter la préparation jusqu'à dissolution complète du Sumatriptan base.

Ajouter 0,450 L d'eau purifiée au mélange, puis 0,84g de Sumatriptan succinate. A l'aide d'un agitateur à hélice, agiter la préparation jusqu'à obtention d'une suspension homogène et dissolution complète.

Filtrer la préparation sur un filtre en polypropylène ou équivalent de 5µm de porosité et répartir la préparation dans des flacons monodoses de 0,75 mL.

### Formulation 7 :

| | | | |
|---|---|---|---|
| - Sumatriptan : | | | 0,50mg |
| introduit sous forme de : | | | |
| | Sumatriptan base | 0,10mg | |
| | Sumatriptan Succinate | 0,56mg | |
| - alcool éthylique 95% V :V : | | | 0,3ml |
| - Eau purifiée : | | | qsp 0,75ml |

Il est à noter que la quantité de sumatriptan dans 0,56mg de sumatriptan succinate est de 0,4mg.

Cet exemple de formulation peut être obtenu par la mise en oeuvre du procédé décrit en suivant pour un lot de 1000 doses, soit 0,75 L.

Dans une cuve en acier inoxydable introduire 0,3 L d'éthanol 95% V/V et 0,10g de Sumatriptan base.

A l'aide d'un agitateur à hélice agiter la préparation jusqu'à dissolution complète du Sumatriptan base.

Ajouter 0,450 L d'eau purifiée au mélange, puis 0,56g de Sumatriptan succinate. A l'aide d'un agitateur à hélice, agiter la préparation jusqu'à obtention d'une suspension homogène et dissolution complète.

Filtrer la préparation sur un filtre en polypropylène ou équivalent de 5µm de porosité et répartir la préparation dans des flacons monodoses de 0,75 mL.

### Formulation 8 :

| | | | |
|---|---|---|---|
| - Sumatriptan : | | | 0,25mg |
| introduit sous forme de : | | | |
| | Sumatriptan base | 0,05mg | |
| | Sumatriptan Succinate | 0,28mg | |
| - alcool éthylique 95% V :V : | | | 0,4ml |
| - Eau purifiée : | | | qsp 0,75ml |

Il est à noter que la quantité de sumatriptan dans 0,25mg de sumatriptan succinate est de 0,2mg.

Cet exemple de formulation peut être obtenu par la mise en oeuvre du procédé décrit en suivant pour un lot de 1000 doses, soit 0,75 L.

Dans une cuve en acier inoxydable introduire 0,4 L d'éthanol 95% V/V et 0,05g de Sumatriptan base.

A l'aide d'un agitateur à hélice agiter la préparation jusqu'à dissolution complète du Sumatriptan base.

Ajouter 0,450 L d'eau purifiée au mélange, puis 0,28g de Sumatriptan succinate. A l'aide d'un agitateur à hélice, agiter la préparation jusqu'à obtention d'une suspension homogène et dissolution complète.

Filtrer la préparation sur un filtre en polypropylène ou équivalent de 5µm de porosité et répartir la préparation dans des flacons monodoses de 0,75 mL.

### Formulation 9 :

| | | | |
|---|---|---|---|
| - Sumatriptan : | | | 2,00mg |
| introduit sous forme de : | | | |
| | Sumatriptan base | 1,6mg | |
| | Sumatriptan Succinate | 0,56mg | |
| - alcool éthylique 95% V :V : | | | 0,4ml |
| - Eau purifiée : | | | qsp 0,75ml |
| - bicarbonate de sodium | | | qs pH 7,5 |

Il est à noter que la quantité de sumatriptan dans 0,56mg de sumatriptan succinate est de 0,4mg.

Cet exemple de formulation peut être obtenu par la mise en oeuvre du procédé décrit en suivant pour un lot de 1000 doses, soit 0,75 L.

Dans une cuve en acier inoxydable introduire 0,4 L d'éthanol 95% V/V et 1,6g de Sumatriptan base.

A l'aide d'un agitateur à hélice agiter la préparation jusqu'à dissolution complète du Sumatriptan base.

Ajouter 0,450 L d'eau purifiée au mélange, puis 0,56g de Sumatriptan succinate. A l'aide d'un agitateur à hélice, agiter la préparation jusqu'à obtention d'une suspension homogène et dissolution complète.

Ajouter du bicarbonate de sodium pour ajuster le pH de la solution à 7,5.

Filtrer la préparation sur un filtre en polypropylène ou équivalent de 5µm de porosité et répartir la préparation dans des flacons monodoses de 0,75 mL.

Une étude pilote a été menée pour monter l'efficacité clinique notamment des formulations 2 et 3, contre la crise aiguë d'Algie Vasculaire de la Face.

L'étude a été menée sur 23 patients.

Les formulations 2 et 3 des exemples ont été administrées aux patients.

La rémission totale de la crise de l'AVF, sédation complète de la pathologie, a été obtenue en moyenne chez 43,5% des patients après 20 minutes. Les résultats obtenus en pourcentage de rémission à 20 minutes sont présentés dans le tableau ci-dessous :

| | **% Rémission à 20 min** |
|---|---|
| **Formulation 2 (4mg)** | 60,0% |
| **Formulation 3 (2mg)** | 41,7% |

On constate que les rémissions les plus importantes à 20 minutes sont obtenues avec la dose à 4mg (60%). En outre la dose 2mg permet en moins de 20 minutes d'atteindre quasiment le taux de rémission de l'Imigrane soluté nasal à 20mg après 2 heures.

Par ailleurs, l'évaluation de la douleur chez les patients présentant une douleur sévère est présentée dans le tableau ci-après :

| | **% douleur sévère à T0** | **% douleur sévère à T20 min** | **Réduction douleur sévère absolue** | **Réduction douleur sévère relatif** |
|---|---|---|---|---|
| **Formulation 2 (4mg)** | 100% | 20% | -80% | -80% |
| **Formulation 3 (2mg)** | 50% | 50% | -42% | -84% |

On constate que la meilleure réduction de la douleur est obtenue pour le dosage à 4mg qui permet de réduire de 80% la douleur du patient.

L'étude a également permis d'évaluer le pourcentage de patients n'ayant plus aucune douleur ou une douleur faible 20 minutes après l'administration :

| | **% aucune douleur ou douleur faible après 20 min** |
|---|---|
| **Formulation 2 (4mg)** | 60,0% |
| **Formulation 3 (2mg)** | 58% |

En outre, aucun effet secondaire n'a été observé.

Bien entendu, l'invention n'est évidemment pas limitée aux exemples représentés et décrits ci-dessus, mais couvre au contraire toutes les variantes.

## Revendications

1. Forme galénique pour l'administration par voie trans-muqueuse buccale d'au moins un principe actif de la famille des Triptans, comprenant au moins :
- ledit principe actif sous forme base et sous forme de sel, et
- une solution hydroalcoolique titrant au moins 15 degrés d'alcool,
ledit principe actif étant présent en état de dissolution stable et complète dans la solution hydroalcoolique, de façon à permettre une absorption rapide dudit principe actif à travers les muqueuses de la cavité buccale et/ou de l'oropharynx.

2. Forme galénique selon la revendication 1, **caractérisée en ce que** la principe actif est présent entre 5 et 95% sous forme base et entre 5 et 95% sous forme de sel.

3. Forme galénique selon la revendication 1 ou 2, **caractérisée en ce que** le principe actif est présent entre 5 et 40% sous forme base et entre 60 et 95% sous forme de sel.

4. Forme galénique selon l'une des revendications 1 à 3, **caractérisée en ce que** le degré d'alcool de la solution hydroalcoolique est compris entre 15 et 95.

5. Forme galénique selon l'une des précédentes revendications **caractérisée en ce que** le degré d'alcool de la solution hydroalcoolique est compris entre 20 et 70.

6. Forme galénique selon l'une des précédentes revendications, **caractérisée en ce que** la solution hydroalcoolique comprend entre 15 et 85% d'alcool et entre 15 et 85% d'eau en masse.

7. Forme galénique selon l'une des précédentes revendications, **caractérisée en ce que** le au moins un principe actif est présent sous forme base et sous forme de Succinate, de Chlorhydrate ou de Sulfate dudit principe actif.

8. Forme galénique selon l'une des précédentes revendications, **caractérisée en ce que** le au moins un principe actif est le Sumatriptan.

9. Forme galénique selon l'une des précédentes revendications, **caractérisée en ce que** la solution hydroalcoolique comprend au moins de l'éthanol.

10. Forme galénique selon l'une des précédentes revendications, **caractérisée en ce que** la solution hydroalcoolique comprend un agent correcteur de pH.

11. Forme galénique selon la revendication 10, **caractérisée en ce que** l'agent correcteur de pH est choisi parmi les carbonates et bicarbonates de sodium, les phosphates monosodique ou disodique, la triéthanolamine, la soude, la potasse et/ou parmi des agents acides Chlorhydrique, Sulfurique, Succinique, Butyrique, Phosphorique, Citrique, Malique et/ou Lactique.

12. Forme galénique selon l'une quelconque des précédentes revendications, **caractérisée en ce que** le pH est compris entre 4,0 et 9,0.

13. Forme galénique selon l'une quelconque des revendications précédentes, **caractérisée en ce que** le volume de solution hydroalcoolique est inférieur à 5 ml.

14. Procédé d'obtention d'une forme galénique selon l'une des revendications 1 à 13, **caractérisé en ce qu'**il comprend les étapes suivantes :
- introduire dans de l'alcool au moins un principe actif de la famille des Triptans sous forme base,
- agiter la préparation jusqu'à dissolution complète dudit principe actif,
- ajouter de l'eau purifiée à la préparation, puis ledit principe actif sous forme de sel,
- agiter la préparation jusqu'à dissolution complète, et
- ajouter de l'eau si nécessaire pour compléter au volume désiré.

15. Procédé d'obtention selon la revendication 14, **caractérisé en ce qu'**il comprend les étapes suivantes :
- introduire dans de l'alcool sous agitation du Sumatriptan base,
- agiter la préparation, préférentiellement durant 10 à 60 minutes, jusqu'à dissolution complète dudit principe actif,
- ajouter de l'eau purifiée à la préparation, puis du Succinate de Sumatriptan,
- agiter la préparation, préférentiellement durant 10 à 60 minutes, jusqu'à dissolution complète,
- ajouter de l'eau si nécessaire pour compléter au volume désiré,
- ajouter des agents correcteurs de pH pour obtenir un pH physiologiquement compatible compris entre 4,0 et 9,0,
- filtrer la préparation, et
- répartir le soluté obtenu dans des contenants adaptés de moins de 5ml de volume.

16. Utilisation de la forme galénique selon l'une des revendications 1 à 13, pour la réalisation d'un médicament destiné au traitement des crises et urgences migraineuses.

## Claims

1. Galenical form for the administration by buccal transmucous means of at least one active ingredient of the triptan family, comprising at least:
- Said active ingredient in basic form and in salt form, and
- A dilute-alcoholic solution that titrates at least 15 degrees of alcohol,
whereby said active ingredient is present in a stable and complete state of dissolution in the dilute-alcoholic solution so as to allow a fast absorption of said active ingredient through the mucous membranes of the buccal cavity and/or the oropharynx.

2. Galenical form according to claim 1, wherein the active ingredient is present between 5 and 95% in basic form and between 5 and 95% in salt form.

3. Galenical form according to claim 1 or 2, wherein the active ingredient is present between 5 and 40% in basic form and between 60 and 95% in salt form.

4. Galenical form according to one of claims 1 to 3, wherein the degree of alcohol of the dilute-alcoholic solution is between 15 and 95.

5. Galenical form according to one of the preceding claims, wherein the degree of alcohol of the dilute-alcoholic solution is between 20 and 70.

6. Galenical form according to one of the preceding claims, wherein the dilute-alcoholic solution comprises between 15 and 85% by mass of alcohol and between 15 and 85% by mass of water.

7. Galenical form according to one of the preceding claims, wherein the - or at least one - active ingredient is present in basic form and in the form of succinate, chlorohydrate, or sulfate of said active ingredient.

8. Galenical form according to one of the preceding claims, wherein the - or at least one - active ingredient is Sumatriptan.

9. Galenical form according to one of the preceding claims, wherein the dilute-alcoholic solution comprises at least ethanol.

10. Galenical form according to one of the preceding claims, wherein the dilute-alcoholic solution comprises a pH-correcting agent.

11. Galenical form according to claim 10, wherein the pH-correcting agent is selected from among the sodium carbonates and bicarbonates, the monosodium or disodium phosphates, triethanolamine, soda, potash and/or from among the agents of hydrochloric acid, sulfuric acid, succinic acid, butyric acid, phosphoric acid, citric acid, malic acid and/or lactic acid.

12. Galenical form according to any of the preceding claims, wherein the pH is between 4.0 and 9.0.

13. Galenical form according to any of the preceding claims, wherein the volume of dilute-alcoholic solution is less than 5 ml.

14. Process for obtaining a galenical form according to one of claims 1 to 13, wherein it comprises the following stages:
- Introducing into the alcohol at least one active ingredient from the family of triptans in basic form,
- Stirring the preparation until said active ingredient is completely dissolved,
- Adding purified water to the preparation, and then said active ingredient in the form of salt,
- Stirring the preparation until dissolution is complete, and
- Adding water if necessary to round out the desired volume.

15. Process for production according to claim 14, wherein it comprises the following stages:
- Introducing basic Sumatriptan into the alcohol while being stirred,
- Stirring the preparation, preferably for 10 to 60 minutes, until said active ingredient is completely dissolved,
- Adding purified water to the preparation, and then Sumatriptan succinate,
- Stirring the preparation, preferably for 10 to 60 minutes, until dissolution is complete,
- Adding water if necessary to round out the desired volume,
- Adding pH-correcting agents to obtain a physiologically compatible pH of between 4.0 and 9.0,
- Filtering the preparation, and
- Distributing the solute that is obtained in suitable containers of less than 5 ml of volume.

16. Use of the galenical form according to one of claims 1 to 13 for the production of a medication that is designed for the treatment of migraine attacks and emergencies.

## Patentansprüche

1. Galenische Form zur oralen transmukosalen Zuführung mindestens eines Wirkstoffes der Familie der Triptane, aufweisend mindestens:
- den Wirkstoff in Grundform und in Form von Salz, und
- eine hydro-alkoholische Lösung mit einem Alkoholgehalt von mindestens 15 Grad,
wobei der Wirkstoff als stabile und vollständige Auflösung in der hydro-alkoholischen Lösung vorliegt, so dass eine schnelle Aufnahme des Wirkstoffs über die Schleimhäute der Mundhöhle und/oder des Mundrachens ermöglicht wird.

2. Galenische Form nach Anspruch 1, **dadurch gekennzeichnet, dass** der Wirkstoff zwischen 5 und 95% in der Grundform und zwischen 5 und 95% in Form von Salz vorliegt.

3. Galenische Form nach Anspruch 1 oder 2, **dadurch gekennzeichnet, dass** der Wirkstoff zwischen 5 und 40% in der Grundform und zwischen 60 und 95% in Form von Salz vorliegt.

4. Galenische Form nach einem der Ansprüche 1 bis 3, **dadurch gekennzeichnet, dass** der Alkoholgehalt der hydro-alkoholischen Lösung zwischen 15 und 95 Grad liegt.

5. Galenische Form nach einem der vorherigen Ansprüche, **dadurch gekennzeichnet, dass** der Alkoholgehalt der hydro-alkoholischen Lösung zwischen 20 und 70 Grad liegt.

6. Galenische Form nach einem der vorhergehenden Ansprüche, **dadurch gekennzeichnet, dass** die hydro-alkoholische Lösung zwischen 15 und 85% Alkohol und zwischen 15 und 85% Wasser in Masse aufweist.

7. Galenische Form nach einem der vorhergehenden Ansprüche, **dadurch gekennzeichnet, dass** der mindestens eine Wirkstoff in Grundform und in Form von Succinat, Hydrochlorid oder Sulfat des Wirkstoffs vorliegt.

8. Galenische Form nach einem der vorhergehenden Ansprüche, **dadurch gekennzeichnet, dass** der mindestens eine Wirkstoff Sumatriptan ist.

9. Galenische Form nach einem der vorhergehenden Ansprüche, **dadurch gekennzeichnet, dass** die hydro-alkoholische Lösung zumindest Ethanol enthält.

10. Galenische Form nach einem der vorhergehenden Ansprüche, **dadurch gekennzeichnet, dass** die hydro-alkoholische Lösung ein pH-Ausgleichsmittel enthält.

11. Galenische Form nach Anspruch 10, **dadurch gekennzeichnet, dass** das pH-Ausgleichsmittel ausgewählt ist aus Natriumcarbonaten und -bicarbonaten, Mononatriumphosphaten oder Dinatriumphosphaten, Triethanolamin, Soda, Pottasche, und/oder aus den Mitteln Salzsäure, Schwefelsäure, Bernsteinsäure, Buttersäure, Phosphorsäure, Zitronensäure, Apfelsäure und/oder Milchsäure.

12. Galenische Form nach einem der vorhergehenden Ansprüche, **dadurch gekennzeichnet, dass** der pH-Wert zwischen 4,0 und 9,0 liegt.

13. Galenische Form nach einem der vorhergehenden Ansprüche, **dadurch gekennzeichnet, dass** das Volumen der hydro-alkoholischen Lösung unter 5 ml liegt.

14. Verfahren zur Gewinnung einer galenischen Form nach einem der Ansprüche 1 bis 13, **dadurch gekennzeichnet, dass** es die folgenden Schritte aufweist:
- Einbringen mindestens eines Wirkstoffes der Familie der Triptane in Grundform in den Alkohol,
- Rühren des Präparats bis zur vollständigen Auflösung des Wirkstoffs,
- Hinzugeben von gereinigtem Wasser zum Präparat, dann des Wirkstoffs in Form von Salz,
- Rühren des Präparats bis zur vollständigen Auflösung, und
- gegebenenfalls Hinzugeben von Wasser, um das gewünschte Volumen zu vervollständigen.

15. Verfahren zur Gewinnung nach Anspruch 14, **dadurch gekennzeichnet, dass** es die folgenden Schritte aufweist:
- Einbringen von Sumatriptan in Grundform in den Alkohol unter Rühren,
- Rühren des Präparats, vorzugsweise während 10 bis 60 Minuten, bis zur vollständigen Auflösung des Wirkstoffs,
- Hinzugeben von gereinigtem Wasser zu dem Präparat, dann von Sumatriptansuccinat,
- Rühren des Präparats, vorzugsweise während 10 bis 60 Minuten, bis zur vollständigen Auflösung,
- gegebenenfalls Hinzugeben von Wasser, um das gewünschte Volumen zu vervollständigen,
- Hinzugeben von pH-Ausgleichsmitteln, um einen physiologisch verträglichen pH-Wert zwischen 4,0 und 9,0 zu erhalten,
- Filtern des Präparats, und
- Verteilen des erhaltenen gelösten Stoffes auf passende Behälter mit einem Volumen von weniger als 5 ml.

16. Verwendung der galenischen Form nach einem der Ansprüche 1 bis 13 zur Verwirklichung eines Medikamentes zur Behandlung von Migräneattacken und -notfällen.
